# EUROPEAN PATENT SPECIFICATION

(11) **EP 2 968 266 B1**
(45) Date of publication and mention of the grant of the patent: **01.05.2019**
(21) Application number: 14770138.7
(22) Date of filing: 11.03.2014
(51) Int. Cl.: A61P 3/06, A61K 31/4015, A61K 31/4045, A61K 31/415, A61K 31/4155, A61K 31/4178, A61K 31/4196, A61K 31/42, A61K 31/426, A61K 31/433, A61K 31/4439, A61K 31/4545, A61K 31/5377

(54) **ANTI-PROPROTEIN CONVERTASE SUBTILISIN KEXIN TYPE 9 (ANTI-PCSK9) COMPOUNDS IN THE TREATMENT AND/OR PREVENTION OF CARDIOVASCULAR DISEASES**
ANTI-PROPROTEINKONVERTASESUBTILISINKEXIN VOM TYP 9 (ANTI-PCSK9)-VERBINDUNGEN BEI DER BEHANDLUNG UND/ODER PRÄVENTION VON KARDIOVASKULÄREN ERKRANKUNGEN
COMPOSÉS ANTI-PROPROTÉINE CONVERTASE SUBTILISINE KEXINE DE TYPE 9 (ANTI-PCSK9) DANS LE TRAITEMENT ET/OU LA PRÉVENTION DE MALADIES CARDIO-VASCULAIRES

(30) Priority: 15.03.2013 US 201361788061 P
(43) Date of publication of application: 20.01.2016
(73) Proprietor: Shifa Biomedical Corporation, Malvern, PA 19355 (US)
(72) Inventor: ABDEL-MEGUID, Sherin Salaheldin, Exton, PA 19341 (US); ELSHOURBAGY, Nabil, West Chester, PA 19380 (US); MEYERS, Harold, Weston, PA 02493 (US); MOUSA, Shaker A., Wynantskill, NY 12198 (US)
(74) Representative: Mewburn Ellis LLP
(86) International application number: PCT/US2014/022957
(87) International publication number: WO 2014/150326

(56) References cited:
- US-A- 4 017 313
- US-A- 4 371 607
- US-A1- 2008 090 829
- US-A1- 2009 275 053
- US-B2- 8 003 654
- YANGTHARA BURANEE ET AL: "Small-molecule vasopressin-2 receptor antagonist identified by a G-protein coupled receptor pathway screen", MOLECULAR PHARMACOLOGY, AMERICAN SOCIETY FOR PHARMACOLOGY AND EXPERIMENTAL THERAPEUTICS, US, vol. 72, no. 1, 1 January 2007 (2007-01-01), pages 86-94, XP002526336, ISSN: 0026-895X, DOI: 10.1124/MOL.107.034496

## Description

### Statement Regarding Federal Sponsored Research Or Development

The present invention was made with support from the National Heart, Lung and Blood Institute (NHLBI) under SBIR Grant No. 1R43HL096167-01. The U.S. Government has certain rights in this invention.

### Field of Invention

The present invention relates to medical uses of compounds that modulate the physiological action of the
proprotein convertase subtilisin kexin type 9 (PCSK9), including its interaction with the low density lipoprotein receptor (LDLR). More specifically, the invention relates to compositions comprising small molecule modulators of PCSK9 function and methods of using these modulators as a medicament. The small molecule modulators of PCSK9 function can be used therapeutically to lower LDL-cholesterol levels in blood, and can be used in the prevention and/or treatment of hypercholesterolemia, and/or at least one symptom of hypercholesterolemia in dyslipidemia, atherosclerosis, CVD or coronary heart disease.

### Background of Invention

Cardiovascular diseases are the leading cause of death, with atherosclerosis being the leading cause of cardiovascular diseases. Atherosclerosis is a disease of the arteries and is responsible for coronary heart disease associated with many deaths in industrialized countries. Several risk factors for coronary heart disease have now been identified: dyslipidemia, hypertension, diabetes, smoking, poor diet, inactivity and stress. Dyslipidemia is elevation of plasma cholesterol (hypercholesterolemia) and/or triglycerides (TGs) or a low high-density lipoprotein (HDL) level that contributes to the development of atherosclerosis. It is a metabolic disorder that is proven to contribute to cardiovascular disease. In the blood, cholesterol is transported in lipoprotein particles, where the low-density lipoprotein (LDL) cholesterol (LDL-C) is considered "bad" cholesterol, while HDL-cholesterol (HDL-C) is known as "good" cholesterol. Lipid and lipoprotein abnormalities are extremely common in the general population and are regarded as a highly modifiable risk factor for cardiovascular disease, due to the influence of cholesterol on atherosclerosis. There is a long-felt significant unmet need with respect to CVD with 60-70% of cardiovascular events, heart attacks and strokes occurring despite the treatment with statins (the current standard of care in atherosclerosis). Moreover, new guidelines suggest that even lower LDL levels should be achieved in order to protect high risk patients from premature CVD (**1**).

The establishment of a link between PCSK9 and cholesterol metabolism was rapidly followed by the discovery that selected mutations in the PCSK9 gene caused autosomal dominant hypercholesterolemia (**2**), suggesting that the mutations confer a gain-of-function (**3**) by increasing the normal activity of PCSK9. This was supported by the experiment in which wild-type and mutant PCSK9 (S127R and F216L) were expressed at high levels in the livers of mice; hepatic LDLR protein levels fell dramatically in mice receiving either the wild-type or mutant PCSK9 (**4, 5**). No associated reductions in LDLR mRNA levels were observed, indicating that overexpression of PCSK9, whether mutant or wild-type, reduces LDLRs through a post-transcriptional mechanism.

Given that gain-of-function mutations in PCSK9 cause hypercholesterolemia, it was reasonable to ask if loss-of-function mutations would have the opposite effect and result in hypocholesterolemia. Three loss-of-function mutations in PCSK9 (Y142X, L253F, and C679X) were identified in African-Americans (**6**). These mutations reduce LDL-C levels by 28% and were shown to decrease the frequency of CHD (defined as myocardial infarction, coronary death or coronary revascularization) by 88%. Rashid et al. (**7**) studied the mechanism of loss-of-function mutations in mice where PCSK9 was inactivated. They reported that these knockout mice showed increased hepatic LDLR protein (but not mRNA), increased clearance of circulating lipoproteins and reduced plasma cholesterol levels. Structure-function relationship analysis of the naturally occurring mutations in PCSK9 has also provided insights into the mechanism of action of PCSK9. Interestingly, mutations in PCSK9 that were found to be associated with the greatest reductions in LDL-C plasma levels are those that prevent the secretion of mature PCSK9 by disrupting its synthesis (Y142X), autocatalytic processing (L253F), or folding (C679X) (**8**). The Y142X mutation produces no detectable protein because it occurs early in the transcript and is predicted to initiate nonsense-mediated mRNA decay. Mutations in the catalytic domain (L253F) interfere with the autocatalytic cleavage of the protein. In cells expressing the PCSK9-253F, the amount of mature protein was reduced compared to that in cells expressing PCSK9-WT, suggesting that the mutation inhibits autocatalytic cleavage. The L253F mutation is near the catalytic triad (PCSK9 is a serine protease), therefore it might disrupt the active site (**8**). Inasmuch as autocatalytic cleavage of PCSK9 is required for export of the protein out of the ER, the L253F mutation delays transport of PCSK9 from the ER to the cell surface. The nonsense mutation (C679X) in PCSK9, which truncates the protein by 14 amino acids, did not interfere with protein processing, but the mature protein accumulates in the cells and none is secreted, suggesting that the protein is cleaved normally but is misfolded and is retained in the ER (**8, 9**).

The mechanism by which PCSK9 causes the degradation of the LDLR has not been fully elucidated. However, it is clear that the protease activity of PCSK9 is not required for LDLR degradation (**10,11**). Li et al. (**10**) have co-expressed the prodomain and the catalytic domain in trans, and showed that the secreted PCSK9 was catalytically inactive, yet it is functionally equivalent to the wild-type protein in lowering cellular LDL uptake and LDLR levels. Similar studies were also reported by McNutt et al. (**11**). Furthermore, Zhang et al. (**12**) has mapped PCSK9 binding to the EGF-A repeat of the LDLR, and showed that such binding decreases the receptor recycling and increases its degradation. They also reported that binding to EGF-A domain was calcium-dependent and increased dramatically with reduction in pH from 7 to 5.2. Recently, Kwon et al. (**13**) determined the crystal structure of PCSK9 in complex with the LDLR-EGF-AB (EGF-A and EGF-B). The structure shows a well defined EGF-A domain, but the EGF-B domain is disordered and absent from their electron density map. The EGF-A domain binds to the PCSK9 catalytic domain at a site distant from the catalytic site, and makes no contact with either the C-terminal domain or the prodomain (**14**).

Several strategies have been proposed for targeting PCSK9 (**15**). Strategy 1: mRNA knockdown approaches include the use of antisense oligonucleotides or RNAi. Antisense oligonucleotides administered to mice reduced PCSK9 expression by >90% and lowered plasma cholesterol levels by 53% (**16**). A single intravenous injection of an RNAi delivered in lipidoid nanoparticles to cynomologous monkeys reduced plasma PCSK9 levels by 70% and plasma LDL-C levels by 56% (**17**). Strategy 2: is to prevent binding of PCSK9 to the LDLR on the cell surface with a small molecule, a peptide, or an antibody directed against PCSK9. Adding EGF-A fragments to cultured cells inhibits the ability of exogenously added PCSK9 to mediate LDLR degradation. Strategy 3: is to develop small-molecule inhibitors of the PCSK9 processing. Despite evidence that the catalytic activity of PCSK9 is not required for LDLR degradation (**11**), an intracellular inhibitor of PCSK9 catalytic activity should be effective, since autocatalytic processing of PCSK9 is required for secretion of the protein from the ER. Following its synthesis, PCSK9 undergoes an autocatalytic cleavage reaction that clips off the prodomain, but the prodomain remains attached to the catalytic domain (**18,19**). The autocatalytic processing step is required for the secretion of PCSK9 (**20**), likely because the prodomain serves as a chaperone and facilitates folding. The continued attachment of the prodomain partially blocks the substrate binding pocket of PCSK9 (**18,19**). McNutt et al. (**21**) demonstrated that antagonism of secreted PCSK9 increases LDLR expression in HepG2 cells. They show that an FH-associated LDLR allele (H306Y) that results in a gain-of-function mutation is due to an increase in the affinity of PCSK9 to the LDLR, which would lead to enhanced LDLR destruction, and decreased plasma LDL-C clearance. Furthermore, they were able to show elegantly that blocking the secreted PCSK9 with LDLR (H306Y) subfragment resulted in an increase in the level of LDLR in cultured HepG2 cells. Therefore, PCSK9 acts as a secreted factor to cause LDLR degradation, and a small molecule inhibitor that interferes with the autocatalytic process should decrease the amount of mature secreted PCSK9. This invention relates to identification of small molecules that down-regulate the function of PCSK9 using Strategy 2.

Recently (**22-24**), Regeneron/Sanofi and Amgen have reported Phase II proof-of-concept data that validate the blocking of PCSK9 with a monoclonal antibody as a strategy for lowering LDL-C in patients not controlled on standard statin therapy. They reported that a single injection of their drug, called REGN727, slashed LDL levels by more than 60% in clinical trial. Their approach follows Strategy 2 using antibodies instead of small molecules. This Strategy 2 is also being pursued by Merck, Novartis and Pfizer, while Strategy 1 is being pursued by Alnylam, Idera and Santaris (25). US2009/275053 describes PCSK9 modulation with nucleotides for the treatment of hypercholesteremia.

### Summary of the Invention

The scope of the present invention is defined by the appended claims. Any other disclosure in the present description, regardless of whether it is indicated as preferred or exemplified, does not form part of the present invention.

In a first aspect, the present invention provides a compound for use in a method for treating or preventing hypercholesterolemia, and/or at least the symptom of hypercholesterolemia in dyslipidemia, atherosclerosis, CVD or coronary heart disease, wherein the compound is represented by the formula:
wherein
R₁ is selected from the group of optionally substituted cycloalkyl, cycloalkyloxy, aryl, aryloxy, aralkoxy, heterocycle, heterocycloalkoxy, heteroaryl, and heteroaralkoxy;
R₂ is selected from the group of H and optionally substituted lower alkyl, cycloalkyl, aryl, heterocycle, and heteroaryl;
R₄ is selected from the group of H, lower alkyl, OH, SH, NH₂, halo, CN, carboxyl, and carboxamido;
wherein X¹, Y¹ and Z¹ are the same or different and each represents hydrogen or a substituent from the group consisting of hydroxyl, halogen, amino, monoalkylamino, dialkylamino, alkoxy, carboxy, amido (including formamido, alkylamido and arylamido), aminocarbonyl, monoalkylaminocarbonyl, dialkylaminocarbonyl, carbamato, carboxamido, monoalkylaminosulfinyl, dialkylaminosulfinyl, monoalkylaminosulfonyl, dialkylaminosulfonyl, alkylsulfonylamino, hydroxysulfonyloxy, alkoxysulfonyloxy, alkylsulfonyloxy, hydroxysulfonyl, alkoxysulfonyl, alkylsulfonylalkyl, monoalkylaminosulfonylalkyl, dialkylaminosulfonylalkyl, monoalkylaminosulfinylalkyl, dialkylaminosulfmylalkyl and, optionally substituted, lower alkyl, alkenyl, alkynyl, cycloalkyl, aryl, heterocycle, and heteroaryl;
   and
s is an integer from 1 to 4;
or the pharmaceutically acceptable salts and all stereoisomers of said compound.

### Description of Drawings and Tables

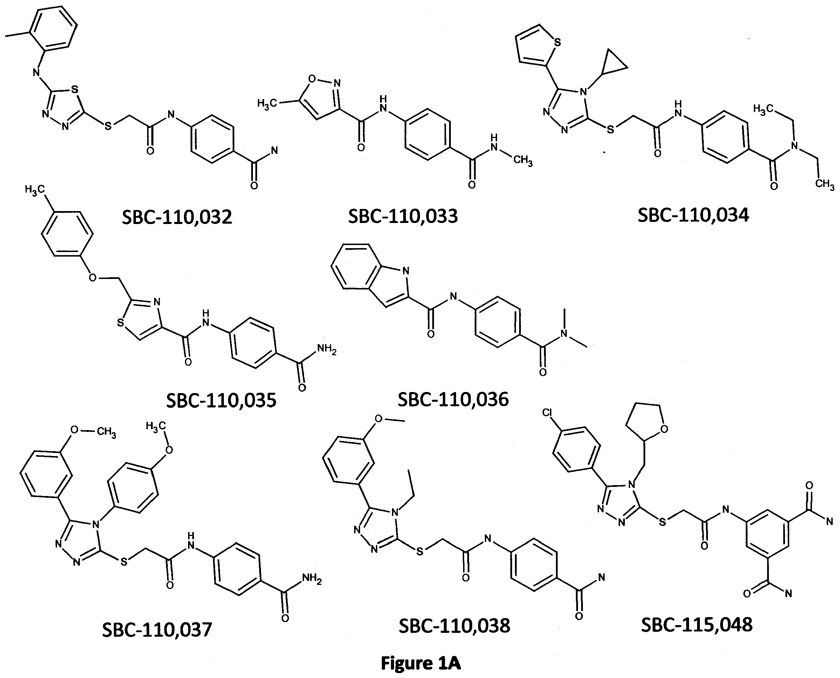

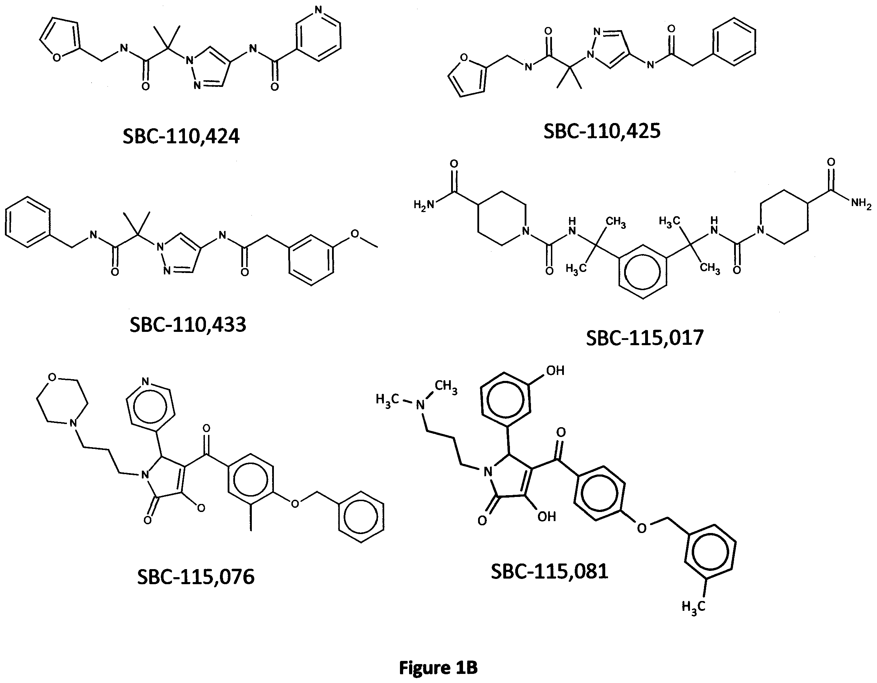

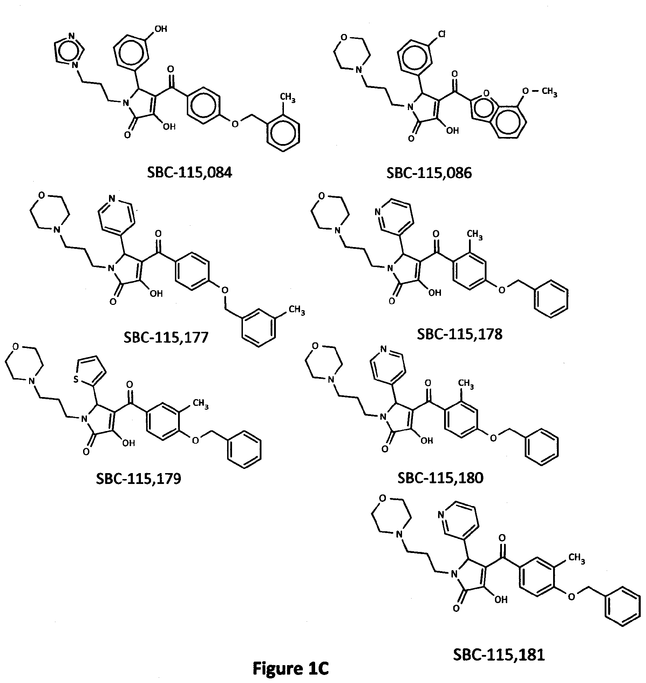

**Figure 1A****, B and C** sets forth the structure of selected compounds that have an effect on LDLR upregulation as compared to control while having no effect on PCSK9 processing and secretion.

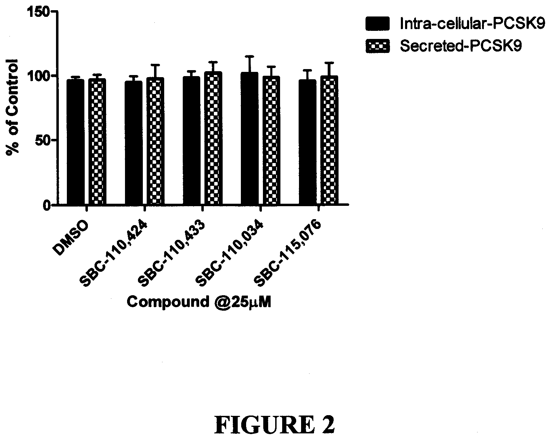

**Figure 2** shows the effect of different hits on PCSK9 synthesis, processing and secretion in HEK293 transfected cells. HEK-293T cells were seeded into 96 well plates in a DMEM containing 10% Fetal Bovine Serum media and incubated overnight at 37°C. Cells were transiently transfected with cDNA construct using the Lipofectamine-LTX. Compounds (25 uM) or vehicle were added, followed by additional 43 hours of incubation. Cellular PCSK9, secreted PCSK9, and cell viability were analyzed as described in Example 1 below.

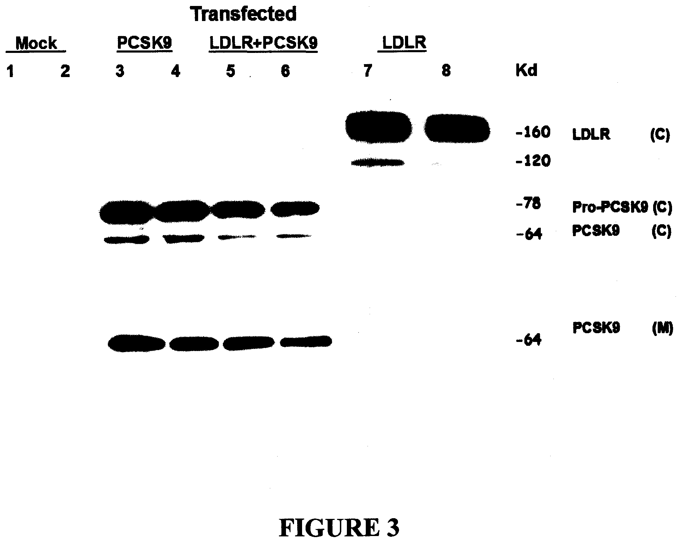

**Figure 3** shows increased degradation of the LDLR by PCSK9. HEK-293T cells were seeded in a DMEM containing 10% Fetal Bovine Serum media and incubated overnight at 37°C. Cells were transiently transfected with Mock (lanes 1 and 2), PCSK9 (lanes 3 and 4), LDLR & PCSK9 (lanes 5 and 6), and LDLR (lanes 7 and 8) cDNA constructs using the Lipofectamine-LTX. Cells were incubated for an additional 72 hrs, and cells and media were analyzed as in text.

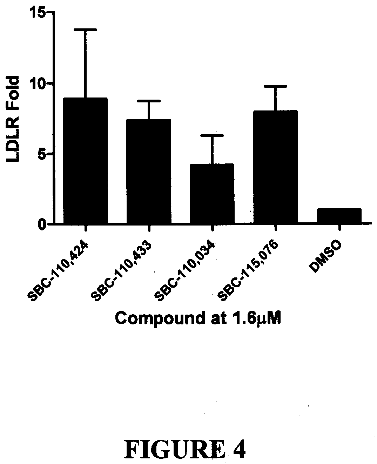

**Figure 4** shows upregulation of LDLR by PCSK9 antagonists. HEK-293T cells were seeded in a DMEM containing 10% Fetal Bovine Serum media and incubated overnight at 37°C. Cells were transiently transfected with LDLR & PCSK9 cDNA constructs using the Lipofectamine-LTX as described above. After 24 hrs, cells were treated with different compounds and incubated for an additional 48 hrs. Cells were assayed as described above for LDLR expression.

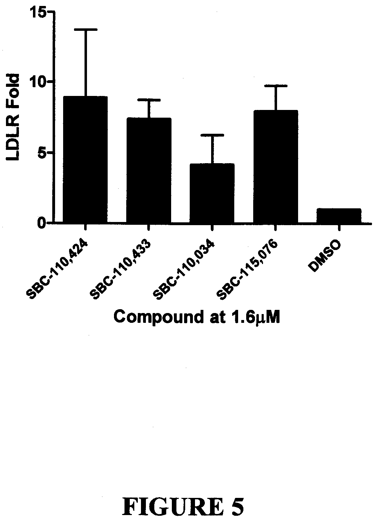

**Figure 5** shows effect of different hits on LDLR upregulation in HepG2 cells. HepG2 cells were seeded into 96 well plates in a MEM containing 10% Fetal Bovine Serum media and incubated overnight at 37°C. Cells were transiently transfected with PCSK9 cDNA constructs using the Lipofectamine-LTX. Compounds were added, followed by additional 43 hours of incubation.
The cells were lysed and analyzed for LDLR expression and cell viability determined as described above.

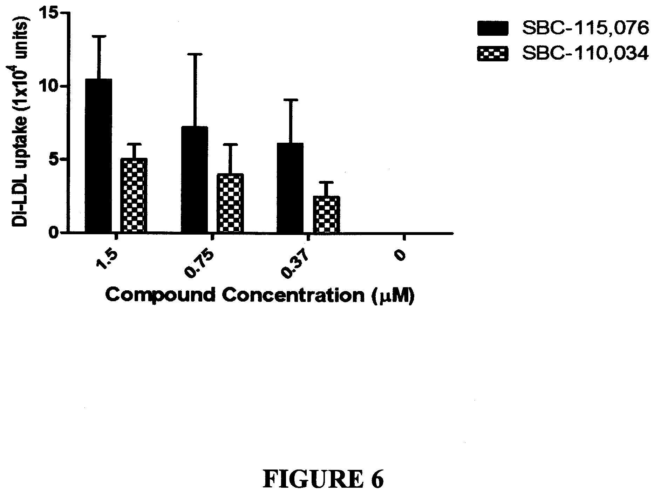

**Figure 6** shows increased uptake of Fluorescent Dil-LDL using various concentrations of two inhibitors in HepG2 cells. The SBC compounds were validated for their ability to increase uptake of Fluorescent Dil-LDL in HepG2 cells. The data show that an increase in the Fluorescent Dil-LDL uptake using submicromolar concentrations of SBC-115,076.

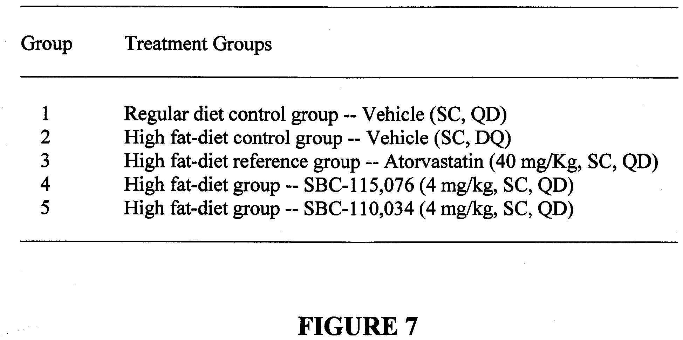

**Figure 7** shows the different treatments for each of the five groups of animals used to test the efficacy of SBC-115,076 and Atorvastatin.

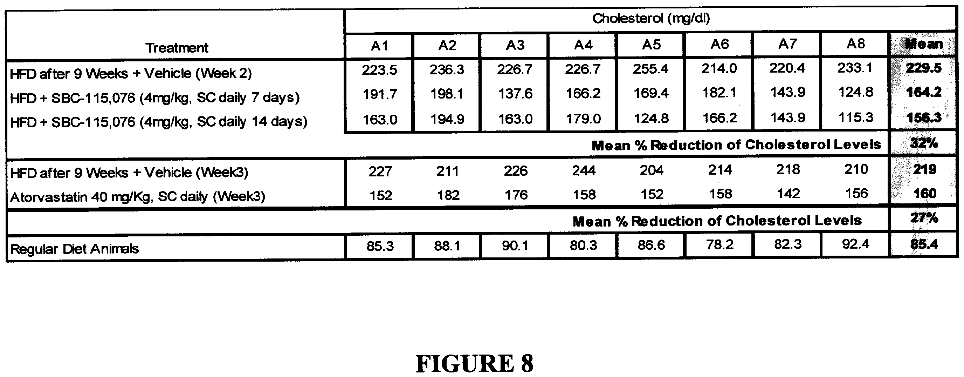

**Figure 8** shows the effect of SBC-115,076 and atorvastatin on serum total cholesterol levels of 8 mice fed high fat diet (HFD) compared to animals fed regular diet.

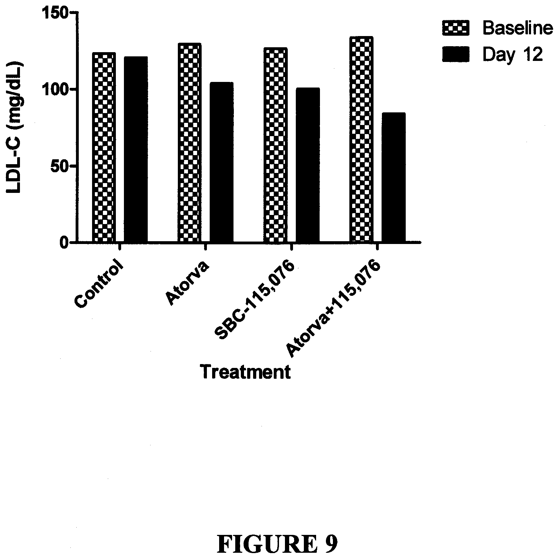

**Figure 9** shows the effect of combinations of SBC-115,076 and atorvastatin on plasma LDL-C in mice fed High Fat Diet.

### Detailed Description of the Invention

The present invention relates to small molecules that down regulate the function of extracellular proprotein convertase subtilisin kexin type 9 (PCSK9), including its interaction with the low density lipoprotein (LDL) receptor (LDLR), and methods of using these antagonists as a medicament. The small molecule modulators of PCSK9 function can be used therapeutically to lower LDL-cholesterol levels in blood, and can be used in the prevention and/or treatment of cholesterol and lipoprotein metabolism disorders, including familial hypercholesterolemia, atherogenic dyslipidemia, atherosclerosis, and, more generally, cardiovascular disease (CVD).

As used herein, the term "lower alkyl" denotes branched or unbranched hydrocarbon chains, having 1 to about 8 carbons, such as, methyl, ethyl, n-propyl, iso-propyl, n-butyl, sec-butyl, isobutyl, tert-butyl, 2-methylpentyl pentyl, hexyl, isohexyl, heptyl, 4,4-dimethyl pentyl, octyl, 2,2,4-trimethylpentyl. "Substituted alkyl" includes an alkyl group optionally substituted with one or more functional groups which are attached commonly to such chains, such as, hydroxy, halogen, mercapto or thio, cyano, alkylthio, carboxy, carbalkoxy, amino, nitro, alkoxy, or optionally substituted, alkenyl, alkynyl, heterocyclyl, aryl, heteroaryl, to form alkyl groups such as trifluoro methyl, 3-hydroxyhexyl, 2-carboxypropyl, 2-fluoroethyl, carboxymethyl, cyanobutyl, phenethyl, benzyl.

The term "halogen" or "halo" as used herein alone or as part of another group refers to chlorine, bromine, fluorine, and iodine.

The term "alkoxy" refers to alkyl-O-, in which alkyl is as defined above.

The term "alkylthio" refers to alkyl-S-, in which alkyl is as defined above.

The terms "amino", "monoalkylamino", "dialkylamino" refer to the moiety -NR'R", in which R' and R", each independently represents H, alkyl or aryl, all as defined herein.

The term "carboxy" refers to the moiety -C(=O)OH.

The term "carbalkoxy" refers to the moiety -C(=O)O-alkyl, in which alkyl is as defined above.

The term "amino (monoalkylamino-, dialkylamino-) carbonylamino" refers to the moiety - NHC(=O)NR'R", in which R'R", each independently represents H, alkyl or aryl, all as defined herein.

The term "carbamato" refers to the moiety -NR'C(=O)OR", in which R' and R", each independently represents H, alkyl or aryl, all as defined herein.

The term "amino (monoalkylamino, dialkylamino) carbonyl" (also "carboxamido") refers to the moiety -C(=O)NR'R", in which R' and R" each independently represents H, alkyl, or aryl, all as defined herein.

The term "amido" refers to the moiety -NRC(=O)-R", in which R' and R", each independently represents H, alkyl or aryl, all as defined herein.

The term "alkylsulfonyl" refers to the moiety -S(=O)2-alkyl, in which alkyl is as previously defined.

The term "alkylsulfonyloxy" refers to the moiety -OS(=O)2-alkyl, wherein alkyl is as previously defined.

The term "amino (monoalkylamino-, dialkylamino-) sulfinyl" refers to the moiety -S(=O)NR'R" in which R' and R" each independently represents H, alkyl or aryl, all as defined herein.

The term "amino (monoalkylamino-, dialkylamino-) sulfonyl" refers to the moiety - S(=O)2NR'R", in which R' and R" each independently represents H, alkyl or aryl, all as defined herein.

The term "alkylsulfonylamino" refers to the moiety NHS(=O)2-alkyl, in which alkyl is as previously defined.

The term "hydroxysulfonyloxy" refers to the moiety -OS(=O)2OH.

The term "alkoxysulfonyloxy" refers to the moiety -OS(=O)2O-alkyl, in which alkyl is as previously defined.

The term "alkylsulfonyloxy" refers to the moiety -OS(=O)2-alkyl, in which alkyl is as previously defined.

The term "hydroxysulfonyl" refers to the moiety -S(=O)2OH.

The term "alkoxysulfonyl" refers to the moiety -S(=O)2O-alkyl, wherein alkyl is as previously defined.

The term "alkylsulfonylalkyl" refers to the moiety -alkyl-S(=O)2-alkyl, wherein alkyl (each instance) is as previously defined.

The term "amino (monoalkylamino-, dialkylamino-) sulfonylalkyl" refers to the moiety -alkyl-S(=O)2-NR'R", wherein alkyl is as previously defined, and R' and R" each independently represents H, alkyl or aryl, all as defined herein.

The term "amino (monoalkylamino-, dialkylamino-) sulfinylalkyl" refer to the moiety -alkyl-S(=O)-NR'R", wherein alkyl is as previously defined, and R' and R" each independently represents H, alkyl or aryl, all as defined herein.

Unless otherwise indicated, the term "cycloalkyl" as employed herein alone or as part of another group includes saturated or partially unsaturated (containing 1 or more double bonds) cyclic hydrocarbon groups containing 1 to 3 rings, including monocyclicalkyl, bicyclicalkyl and tricyclicalkyl, containing a total of 3 to 20 carbons forming the rings, preferably 3 to 10 carbons, forming the ring and which may be fused to 1 or 2 aromatic rings as described for aryl, which include cyclopropyl, cyclobutyl, cyclopentyl, cyclohexyl, cycloheptyl, cyclooctyl, cyclodecyl cyclododecyl and cyclohexenyl,
"Substituted cycloalkyl" includes a cycloalkyl group optionally substituted with 1 or more substituents such as halogen, alkyl, substituted alkyl, alkoxy, hydroxy, aryl, substituted aryl, aryloxy, cycloalkyl, alkylamido, alkanoylamino, oxo, acyl, arylcarbonylamino, amino, nitro, cyano, thiol and/or alkylthio and/or any of the substituents included in the definition of "substituted alkyl."

Unless otherwise indicated, the term "alkenyl" as used herein by itself or as part of another group refers to straight or branched chain of 2 to 20 carbons, preferably 2 to 12 carbons, and more preferably 2 to 8 carbons in the normal chain, which include one or more double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, 4,8,12-tetradecatrienyl. "Substituted alkenyl" includes an alkenyl group optionally substituted with one or more substituents, such as the substituents included above in the definition of "substituted alkyl" and "substituted cycloalkyl."

Unless otherwise indicated, the term "alkynyl" as used herein by itself or as part of another group refers to straight or branched chain of 2 to 20 carbons, preferably 2 to 12 carbons and more preferably 2 to 8 carbons in the normal chain, which include one or more triple bonds in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl, 3-undecynyl, 4-dodecynyl and the like. "Substituted alkynyl" includes an alkynyl group optionally substituted with one or more substituents, such as the substituents included above in the definition of "substituted alkyl" and "substituted cycloalkyl."

Unless otherwise indicated, the term "aryl" or "Ar" as employed herein alone or as part of another group refers to monocyclic and polycyclic aromatic groups containing 6 to 10 carbons in the ring portion (such as phenyl or naphthyl including 1-naphthyl and 2-naphthyl) and may optionally include one to three additional rings fused to a carbocyclic ring or a heterocyclic ring, such as aryl, cycloalkyl, heteroaryl or cycloheteroalkyl rings or substituted forms thereof.

"Substituted aryl" includes an aryl group optionally substituted with one or more functional groups, such as halo, alkyl, haloalkyl (e.g., trifluoromethyl), alkoxy, haloalkoxy (e.g., difluoromethoxy), alkenyl, alkynyl, cycloalkyl-alkyl, cycloheteroalkyl, cycloheteroalkylalkyl, aryl, heteroaryl, arylalkyl, aryloxy, aryloxyalkyl, arylalkoxy, alkoxycarbonyl, alkylcarbonyl, arylcarbonyl, arylalkenyl, aminocarbonyl, monoalkylaminocarbonyl, dialkylaminocarbonyl, aminocarbonylaryl, arylthio, arylsulfinyl, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are optionally substituted alkyl, aryl or any of the other substituents mentioned in the definitions), thiol, alkylthio, arylthio, heteroarylthio, arylthioalkyl, alkoxyarylthio, alkylaminocarbonyl, arylaminocarbonyl, aminocarbonyl, alkylcarbonyloxy, arylcarbonyloxy, alkylcarbonylamino, arylcarbonylamino, arylsulfinyl, arylsulfinylalkyl, arylsulfonylamino or arylsulfonaminocarbonyl and/or any of the alkyl substituents set out herein.

Unless otherwise indicated, the term "heteroaryl" or "Het" as used herein alone or as part of another group refers to a 5- or 7-membered aromatic ring which includes 1, 2, 3 or 4 hetero atoms such as nitrogen, oxygen or sulfur and such rings fused to an aryl, cycloalkyl, heteroaryl or heterocycloalkyl ring (e.g. benzothiophenyl, indolyl), and includes possible N-oxides. "Substituted heteroaryl" includes a heteroaryl group optionally substituted with 1 to 4 substituents, such as the substituents included above in the definition of "substituted alkyl" "substituted cycloalkyl" and "substituted aryl". Substituted heteroaryl also includes fused heteroaryl groups which include, for example, quinoline, isoquinoline, indole, isoindole, carbazole, acridine, benzimidazole, benzofuran, isobenzofuran, benzothiophene, phenanthroline, purine.

The term "heterocyclo", "heterocycle" or "heterocyclic ring," as used herein alone or as part of another group, represents an unsubstituted or substituted stable 5- to 7-membered monocyclic ring system which may be saturated or partially unsaturated, and which consists of carbon atoms and from one to four heteroatoms selected from N, O or S, and wherein the nitrogen and sulfur heteroatoms may optionally be oxidized, and the nitrogen heteroatom may optionally be quaternized. "Substituted heterocyclo (or heterocycle or heterocyclic ring) includes a heterocyclic group optionally substituted with 1 to 4 substituents, such as the substituents included above in the definition of "substituted alkyl" "substituted cycloalkyl" and "substituted aryl". The heterocyclic ring may be attached at any heteroatom or carbon atom which results in the creation of a stable structure. Examples of such heterocyclic groups include, piperidinyl, piperazinyl, oxopiperazinyl, oxopiperidinyl, oxopyrrolidinyl, oxoazepinyl, azepinyl, pyrrolyl, pyrrolidinyl, furanyl, thienyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isooxazolyl, isoxazolidinyl, morpholinyl, thiazolyl, thiazolidinyl, isothiazolyl, thiadiazolyl, tetrahydropyranyl, thiamorpholinyl, thiamorpholinyl sulfoxide, thiamorpholinyl sulfone, and oxadiazolyl.

The term "optionally substituted" is used herein to signify that a chemical moiety referred to, e.g., alkyl, aryl, heteroaryl, may be unsubstituted or substituted with one or more groups including, without limitation, lower alkyl, alkenyl, alkynyl, cycloalkyl, arylalkyl, aryl, haloaryl, heterocycle, heterocycloalkyl, heteroaryl, hydroxyl, amino, monoalkylamino, dialkylamino, alkoxy, halogen, haloalkoxy, aryloxy, aryloxyalkyl, alkylaryloxy, arylalkoxy, alkoxyaryl, carboxy, carbalkoxy, carboxamido, aminocarbonyl, monoalkylaminocarbonyl, dialkylaminocarbonyl, monoalkylaminosulfinyl, dialkylaminosulfinyl, monoalkylaminosulfonyl, dialkylaminosulfonyl, alkylsulfonylamino, hydroxysulfonyloxy, alkoxysulfonyloxy, alkylsulfonyloxy, hydroxysulfonyl, alkoxysulfonyl, alkylsulfonylalkyl, monoalkylaminosulfonylalkyl, dialkylaminosulfonylalkyl, monoalkylaminosulfinylalkyl, dialkylaminosulfinylalkyl. The chemical moieties of formulas I-V, above, that may be optionally substituted include lower alkyl, alkenyl, alkynyl, cycloalkyl, arylalkyl, aryl, heterocycle, and heteroaryl. For example, optionally substituted alkyl would comprise both propyl and 2-chloro-propyl. Additionally, "optionally substituted" is also inclusive of embodiments where the named substituent or substituents have multiple substituents rather than simply a single substituent. For example, optionally substituted aryl would comprise both phenyl and 3-bromo-4-chloro-6-ethyl-phenyl.

As used herein, the term "subject" includes both humans and animals. As used herein, the term "PCSK9" refers to any form of the protein PCSK9, including PCSK9 mutants and variants, which retain at least part of PCSK9 activity or function. Unless otherwise indicated, such as by specific reference to human PCSK9, PCSK9 refers to all mammalian species of native sequence PCSK9, e.g., human, porcine, bovine, equine, canine and feline. One exemplary human PCSK9 sequence is found as Uniprot Accession Number Q8NBP7 (SEQ ID NO: 16).

As used herein, a "modulator of PCSK9 function" refers to a small molecule that is able to inhibit PCSK9 biological activity or function, and/or downstream pathway(s) mediated by PCSK9 signaling, including PCSK9-mediated down-regulation of the LDLR, and PCSK9-mediated inhibition of the decrease in LDL blood clearance. A modulator of PCSK9 function encompasses compounds that block, antagonize, suppress or reduce (to any degree including significantly) PCSK9 biological activity, including downstream pathways mediated by PCSK9 signaling, such as LDLR interaction and/or elicitation of a cellular response to PCSK9. For purpose of the present invention, it will be explicitly understood that the term "modulator of PCSK9 function" encompasses all the previously identified terms, titles, and functional states and characteristics whereby the PCSK9 itself, a PCSK9 biological activity (including its ability to mediate any aspect of interaction with the LDLR, down regulation of LDLR, and inhibit the decrease in blood LDL clearance), or the consequences of the biological activity, are substantially nullified, decreased, or neutralized in any measurable degree. In some embodiments, a modulator of PCSK9 function binds PCSK9 and prevents its interaction with the LDLR or its secretion. In other embodiments, a modulator of PCSK9 function binds to the active site of PCSK9 to stabilize its zymogen and prevent autoprocessing. In further embodiments, a modulator of PCSK9 function decreases or blocks PCSK9 mediated down-regulation of the LDLR; inhibits the PCSK9-mediated decrease in LDL blood clearance; increases LDL clearance in media by cultured hepatocytes; increases blood LDL clearance by the liver *in vivo*; improves patients' sensitivity to other LDL lowering drugs, including statins; is synergistic to other LDL lowering drugs, including statins; and blocks PCSK9 interaction with other yet to be identified factors. Examples of modulators of PCSK9 function are provided herein.

The compounds used in the method of the invention can be administered as salts, which are also within the scope of this invention. Pharmaceutically acceptable (i.e., non-toxic, physiologically compatible) salts are preferred. If the compounds of the method of the present invention have, for example, at least one basic center, they can form acid addition salts. These are formed, for example, with strong inorganic acids, such as mineral acids, for example sulfuric acid, phosphoric acid or a hydrohalic acid, with strong organic carboxylic acids, such as alkanecarboxylic acids of 1 to 4 carbon atoms which are unsubstituted or substituted, for example, by halogen, for example acetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, for example aspartic or glutamic acid or lysine or arginine, or benzoic acid, or with organic sulfonic acids, such as (C1 -C4) alkyl or arylsulfonic acids which are unsubstituted or substituted, for example by halogen, for example methyl- or para-toluene-sulfonic acid. Corresponding acid addition salts can also be formed having plural basic centers, if desired. The compounds used in the method of the present invention having at least one acid group (for example COOH) can also form salts with suitable bases. Representative examples of such salts include metal salts, such as alkali metal or alkaline earth metal salts, for example sodium, potassium or magnesium salts, or salts with ammonia or an organic amine, such as morpholine, thiomorpholine, piperidine, pyrrolidine, a mono, di or tri-lower alkylamine, for example ethyl, tert-butyl, diethyl, diisopropyl, triethyl, tributyl or dimethyl-propylamine, or a mono, di or trihydroxy lower alkylamine, for example mono, di or triethanolamine. Corresponding internal salts may also be formed.

Preferred salts of the compounds described herein which contain a basic group include monohydrochloride, hydrogensulfate, methanesulfonate, phosphate or nitrate.

Preferred salts of the compounds described herein which contain an acid group include sodium, potassium and magnesium salts and pharmaceutically acceptable organic amines.

All stereoisomers of the compounds which may be used in the methods described herein, either in a mixture or in pure or substantially pure form, are considered to be within the scope of this invention. The compounds of the present invention can have asymmetric centers at any of the carbon atoms including any one of the R substituents. Consequently, compounds used in the method of the invention can exist in enantiomeric or diastereomeric forms or in mixtures thereof. The processes for preparation of such compounds can utilize racemates, enantiomers or diastereomers as starting materials. When diastereomeric or enantiomeric products are prepared, they can be separated by conventional methods for example, chromatographic, chiral HPLC or fractional crystallization.

As used herein, the term "pharmacophore" refers to the ensemble of steric and electronic features that are necessary to ensure the optimal supramolecular interactions with a specific biological target structure and to trigger, activate, block, inhibit or modulate the biological target's biological activity, as the case may be. See, IUPAC, Pure and Applied Chemistry (1998) 70: 1129-1143.

As used herein, the term "pharmacophore model" refers to a representation of points in a defined coordinate system wherein a point corresponds to a position or other characteristic of an atom or chemical moiety in a bound conformation of a ligand and/or an interacting polypeptide, protein, or ordered water molecule. An ordered water molecule is an observable water in a model derived from structural determination of a polypeptide or protein. A pharmacophore model can include, for example, atoms of a bound conformation of a ligand, or portion thereof. A pharmacophore model can include both the bound conformations of a ligand, or portion thereof, and one or more atoms that interact with the ligand and are from a bound polypeptide or protein. Thus, in addition to geometric characteristics of a bound conformation of a ligand, a pharmacophore model can indicate other characteristics including, for example, charge or hydrophobicity of an atom or chemical moiety. A pharmacophore model can incorporate internal interactions within the bound conformation of a ligand or interactions between a bound conformation of a ligand and a polypeptide, protein, or other receptor including, for example, van der Waals interactions, hydrogen bonds, ionic bonds, and hydrophobic interactions. A pharmacophore model can be derived from two or more bound conformations of a ligand.

As used herein, the term "ligand" refers to any compound, composition or molecule that interacts with the ligand binding domain of a receptor and modulates its activity. A "ligand" may also include compounds that modulate the receptor without binding directly to it.

In carrying out the method of the invention, the above-described compounds may be administered as such, or in a form from which the active agent can be derived, such as a prodrug. A prodrug is a derivative of a compound described herein, the pharmacologic action of which results from the conversion by chemical or metabolic processes in vivo to the active compound. The term "prodrug esters" as employed herein includes esters and carbonates formed by reacting one or more hydroxyls of compounds used in the method of the invention with alkyl, alkoxy, or aryl substituted acylating agents employing procedures known to those skilled in the art to generate acetates, pivalates, methylcarbonates, benzoates. Any compound that can be converted *in vivo* to provide the bioactive agent (i.e., a compound of formula I) is a prodrug within the scope and spirit of the invention. Various forms of prodrugs are well known in the art. A comprehensive description of prodrugs and prodrug derivatives are described in: (a) The Practice of Medicinal Chemistry, Camille G. Wermuth et al., Ch 31 (Academic Press, 1996); (b) Design of Prodrugs, edited by H. Bundgaard, (Elsevier, 1985); (c) A Textbook of Drug Design and Development, P. Krogsgaard-Larson and H. Bundgaard, eds., Ch. 5, pgs, 113-191 (Harwood Academic Publishers, 1991).

The therapeutic agent used in practicing the method of the invention is administered in an amount sufficient to induce the desired therapeutic effect in the recipient thereof. Thus the term "therapeutically effective amount" as used herein refers to an amount of a therapeutic agent which is sufficient to treat or prevent a condition treatable by administration of one or more of the compounds of formulas I-V or a prodrug thereof. Preferably, the therapeutically effective amount refers to the amount appropriate to treat a PCSK9-associated condition, i.e. to bring almost a detectable therapeutic or preventative or ameliorative effect. The effect may include, for example, treatment or prevention of the conditions described herein.

The compound(s) described herein may be administered at a dose in range from about 0.01 mg to about 200 mg/kg of body weight per day. A dose of from 0.1 to 100, and preferably from 1 to 30 mg/kg per day in one or more applications per day should be effective to produce the desired result. By way of example, a suitable dose for oral administration would be in the range of 1-30 mg/kg of body weight per day, whereas a typical dose for intravenous administration would be in the range of 1-10 mg/kg of body weight per day. Of course, as those skilled in the art will appreciate, the dosage actually administered will depend upon the condition being treated, the age, health and weight of the recipient, the type of concurrent treatment, if any, and the frequency of treatment. Moreover, the effective dosage amount may be determined by one skilled in the art on the basis of routine empirical activity testing to measure the bioactivity of the compound(s) in a bioassay, and thus establish the appropriate dosage to be administered.

The compounds used in the method of the invention will typically be administered from 1-4 times a day, so as to deliver the above-mentioned daily dosage. However, the exact regimen for administration of the compounds described herein will necessarily be dependent on the needs of the individual subject being treated, the type of treatment administered and the judgment of the attending medical specialist.

In one aspect, the invention provides a method for treating or preventing hypercholesterolemia, and/or at least one symptom of dyslipidemia, atherosclerosis, CVD or coronary heart disease, in an individual comprising administering to the individual an effective amount of a modulator of PCSK9 function that antagonizes circulating PCSK9.

In a further aspect, the invention provides an effective amount of a modulator of PCSK9 function that antagonizes circulating PCSK9 for use in treating or preventing hypercholesterolemia, and/or at least one symptom of dyslipidemia, atherosclerosis, CVD or coronary heart disease, in an individual. The invention further provides the use of an effective amount of a modulator of PCSK9 function that antagonizes extracellular or circulating PCSK9 in the manufacture of a medicament for treating or preventing hypercholesterolemia, and/or at least one symptom of dyslipidemia, atherosclerosis, CVD or coronary heart disease, in an individual.

The methods of the invention use a modulator of PCSK9 function, which refers to any molecule that blocks, suppresses or reduces (including significantly reduces) PCSK9 biological activity, including downstream pathways mediated by PCSK9 signaling, such as elicitation of a cellular response to PCSK9.

A modulator of PCSK9 function should exhibit any one or more of the following characteristics: (a) bind to PCSK9; (b) decrease or block PCSK9 interaction with the LDLR; (c) decrease or block secretion of PCSK9; (d) decrease or block PCSK9 mediated down-regulation of the LDLR; (e) inhibit the PCSK9-mediated decrease in LDL blood clearance, (f) increase LDL clearance in media by cultured hepatocytes, (g) increase blood LDL clearance by the liver *in vivo*, (h) improve patients' sensitivity to other LDL lowering drugs, including statins, (i) is synergistic to other LDL lowering drugs, including statins; and (j) block PCSK9 interaction with other yet to be identified factors.

In general, the compound(s) used in the method of the invention can be administered to achieve modulation of PCSK9 function by using any acceptable route known in the art, either alone or in combination with one or more other therapeutic agents. Thus, the active agent(s) can be administered orally, buccally, parenterally, such as by intravenous or intra-arterial infusion, intramuscular, intraperitoneal, intrathecal or subcutaneous injection, by liposome-mediated delivery, rectally, vaginally, by inhalation or insufflation, transdermally or by otic delivery.

The orally administered dosage unit may be in the form of tablets, caplets, dragees, pills, semisolids, soft or hard gelatin capsules, aqueous or oily solutions, emulsions, suspensions or syrups. Suitable dosage forms for parenteral administration include injectable solutions or suspensions, suppositories, powder formulations, such as microcrystals or aerosol spray. The active agent may also be incorporated into a conventional transdermal delivery system.

As used herein, the expression "physiologically compatible carrier medium" includes any and all solvents, diluents, or other liquid vehicle, dispersion or suspension aids, surface agent agents, isotonic agents, thickening or emulsifying agents, preservatives, solid binders, lubricants, fillers and the like as suited for the particular dosage form desired. Remington: The Science and Practice of Pharmacy, 20th edition (A.R. Genaro et al., Part 5, Pharmaceutical Manufacturing, pp. 669-1015 (Lippincott Williams & Wilkins, Baltimore, MD/Philadelphia, PA) (2000)) discloses various carriers used in formulating pharmaceutical compositions and known techniques for the preparation thereof. Except insofar as any conventional pharmaceutical carrier medium is incompatible with the PCSK9 modulators used in the present invention, such as by producing an undesirable biological effect or otherwise interacting in an deleterious manner with any other component(s) of a formulation comprising such compounds, its use is contemplated to be within the scope of this invention.

For the production of solid dosage forms, including hard and soft capsules, the therapeutic agent may be mixed with pharmaceutically inert, inorganic or organic excipients, such as lactose, sucrose, glucose, gelatine, malt, silica gel, starch or derivatives thereof, talc, stearic acid or its salts, dried skim milk, vegetable, petroleum, animal or synthetic oils, wax, fat, polyols, and the like. For the production of liquid solutions, emulsions or suspensions or syrups one may use excipients such as water, alcohols, aqueous saline, aqueous dextrose, polyols, glycerine, lipids, phospholipids, cyclodextrins, vegetable, petroleum, animal or synthetic oils. For suppositories one may use excipients, such as vegetable, petroleum, animal or synthetic oils, wax, fat and polyols. For aerosol formulations, one may use compressed gases suitable for this purpose, such as oxygen, nitrogen and carbon dioxide. The pharmaceutical composition or formulation may also contain one or more additives including, without limitation, preservatives, stabilizers, e.g., UV stabilizers, emulsifiers, sweeteners, salts to adjust the osmotic pressure, buffers, coating materials and antioxidants.

The present invention further includes controlled-release, sustained-release, or extended-release therapeutic dosage forms for administration of the active agent, which involves incorporation of the active agent into a suitable delivery system. This dosage form controls release of the active agent(s) in such a manner that an effective concentration of the active agent(s) in the bloodstream may be maintained over an extended period of time, with the concentration in the blood remaining relatively constant, to improve therapeutic results and/or minimize side effects. Additionally, a controlled-release system would provide minimum peak to trough fluctuations in blood plasma levels of the active agent.

In pharmaceutical compositions used in practicing the method of the invention, the active agent(s) may be present in an amount of at least 0.5 and generally not more than 95% by weight, based on the total weight of the composition, including carrier medium and/or supplemental active agent(s), if any. Preferably, the proportion of active agent(s) varies between 30-90% by weight of the composition.

Preferred compounds for use in practicing this invention include those of formulas II-V, above. More preferred are the compounds set out in Fig. 1.

The compounds described herein are obtainable from commercial sources, such as BioFocus/Galapagos (SBC-110,424, 110,425 and 110,433), Enamine (SBC-110,032 through 038), and Ambinter (SBC-115,017, 115,048 and 115,076, 115,081, 115,084,115,086, and 115,177 through 181).

The uses of the present invention will normally include medical follow-up to determine the therapeutic or prophylactic effect brought about in the subject undergoing treatment with the compound(s) and/or composition(s) described herein.

The activities of compounds described herein have been experimentally demonstrated. The following examples are provided to describe the invention in further detail. These examples are provided for illustrative purposes only.

### Example 1

### Test for Secreted PCSK9

HEK-293T cells were seeded into 96-well plates in a DMEM containing 10% Fetal Bovine Serum media and incubated overnight at 37°C. Cells were transiently transfected with cDNA construct using the Lipofectamine-LTX. Compounds (25 uM) or vehicle were added, followed by additional 43 hours of incubation. Cellular PCSK9, secreted PCSK9, and cell viability were analyzed for PCSK9 secretion using western blot analysis, imaged and quantitated using a LAS-4000 (GE). Results from selected compounds are shown in Figure 2.

### Example 2

### Test for LDLR Upregulation

We used our own recombinant assay which demonstrates that co-expression of PCSK9 and LDLR DNA in HEK-293 cells results in a decrease in the expression level of intracellular LDLRs. We have constructed the expression vector of human LDLR under the control of the cytomegalovirus promoter-enhancer (pCMV-LDLR). In addition, a construct containing the PCSK9 (pCMV-PCSK9-FLAG) was described above. These constructs were used to transiently transfect mammalian cells and both cell lysate and supernatant were subjected to SDS-PAGE and immunoblot analysis using an anti-PCSK9 or LDLR antibody. The data from the blot showed that cells that were transfected with only pCMV-PCSK9-FLAG expressed both the unprocessed (cells) and processed (media) PCSK9 (Figure 3). Cells that were transfected with only pCMV-LDLR showed expression of the LDLR in the cells (Figure 3). However, cells that were transfected with both pCMV-PCSK9-FLAG and pCMV-LDLR showed disappearance of the intracellular LDLR band (Figure 3), which provides further evidence that the presence of PCSK9 results in degradation of LDLR or chaperones it to the degradation pathway. Addition of inhibitors of PCSK9 processing to the latter cells should result in decreased degradation of the LDLR and the appearance of the 160K Dalton band on the gel. Using this assay, we tested our compounds for their ability to reduce the degradation of the LDLR. HEK-293 cells were used in this assay. They were grown in 96-well plates overnight, and transfected with LDLR/PCSK9. Compounds dissolved in DMSO or vehicle were added to the culture media, and incubated for 24-48 hours; cells were lysed. Cell lysates were subjected to quantitation using the above immunoassay (Figure 4).

Testing confirmed that these compounds are capable of up regulating the endogenously expressed LDLR in HepG2 cells. HepG2 transfected with PCSK9 cells were cultured in 96-well plates at a density of 30,000 cells per well. The next day, cells are treated with selected screening compounds or vehicle. Cells were incubated for 48 hrs and then subjected to quantitation using an LDL receptor- polyclonal antibody and analyzed as described above. The data in Figure 5 shows that these compounds exhibited an increase in the level of LDLR as compared to cells treated with same volume of DMSO with several fold upregulation of LDLR

### Example 3

### Uptake of Dil-LDL in HepG2 cells in situ

We also tested the ability of the PCSK9 compounds to enhance the uptake of Fluorescent Dil-LDL in HepG2 cells. Briefly, HepG2 cells were plated and allowed to grow overnight. Compounds were added to the cells followed by the addition of Fluorescent Dil-LDL. Cells were washed extensively, and the Fluorescent Di-LDL taken up by the cells were measured using the Synergy 2 plate reader (Figure 6).

### Example 4

### Test for Cell Viability

All compounds that inhibit PCSK9 secretion were used to test for *in situ* cell viability. HEK-293T cells or HepG2 cells were seeded in 96-well plates in a cell media containing 10% Fetal Bovine Serum and incubated overnight at 37°C. Compounds at various concentrations were added to cells after 24 hours and incubated for an additional 48 hours. Cell viability was assayed using Resazurin (Sigma 199303) and an Envision 2101 Multi-label plate reader.

While certain embodiments of the present invention have been described and/or exemplified above, various other embodiments will be apparent to those skilled in the art from the foregoing disclosure. The term "comprising" is intended to be inclusive or open-ended and does not exclude any additional, unrecited element, method, step or material. The term "consisting of' excludes any element, step or material other than those specified in the claim and, in the latter instance, impurities ordinarily associated with the specified material(s).

### Example 5

### Test for Efficacy in Animal Model

SBC- 115,076, and SBC-110,034 (reference compound) were tested for their efficacy in male mice (C57BL/6 mice). Mice were housed as four animals per cage under climate-controlled conditions of temperature (20-24°C), humidity (60-70%), and alternating 12h light/dark cycles. The mice were divided into five groups as shown in Figure 7. One group was fed commercial chow diet (Prolab RMH 3000, PMI feeds, St. Louis, MO) to serve as a negative control, while the other four groups were fed high fat diet (TD.06414), which provides 60% of calories from fat. Water was provided ad libitum. Plasma was collected once weekly to monitor the level of LDL. After 4 weeks of feeding a high fat-diet, mice were randomly assigned to one of several groups such that the average LDL levels were equal among different groups. One of the four groups of mice fed high fat diet was treated with vehicle and served as a positive control, whereas each of the other three groups was treated daily with 8 mg/kg of one of the above-mentioned compounds subcutaneously for two weeks. Blood samples (75 µl) were collected twice weekly after drug administration from the retro-orbital venous plexus via heparinized capillary tubes containing 2 USP units of ammonium heparin per tube (Carolina, Burlington, NC). Plasma were separated immediately by centrifugation (5,000 x g) for 5 min at room temperature and then kept at -80°C until assayed for lipid profile. Plasma cholesterol, LDL-C, HDL-C, and triglyceride levels are measured enzymatically. Additionally, the plasma levels of PCSK9 and chemokines / cytokines were measured for potential pleiotropic effects of PCSK9 inhibitors using ELISA and multiplex assays.

The data obtained demonstrated that SBC-115,076 and SBC-110,034 lowered cholesterol levels in mice that are fed high fat diet. Figure 8 shows data obtained with SBC-115,076 indicating a mean of 32% reduction (P < 0.01) in total cholesterol levels after two weeks relative to high fat diet animal levels and a mean 50% reduction (P < 0.01) toward return to regular diet cholesterol levels.

A second study was conducted with Atorvastatin (40 mg/kg, for 3 weeks). The data demonstrated that with Atorvastatin a mean of 27% reduction (P < 0.01) in total cholesterol levels after three weeks relative to high fat diet animal levels and a mean 44% reduction (P < 0.01) toward return to regular diet cholesterol levels (Figure 9).

### References

1. Grundy SM, Cleeman JI, Merz CNB, Brewer, Jr, HB, Clark LT, Hunninghake DB, Pasternak RC, Smith, Jr, SC, Stone NJ (2004). Implications of Recent Clinical Trials for the National Cholesterol Education Program Adult Treatment Panel III Guidelines. Circulation 110, 227-239.
2. Abifadel M, Varret M, Rabès J, Allard D, Ouguerram K, Devillers M, Cruaud C, Benjannet S, Wickham L, Erlich D, Derré A, Villéger L, Farnier M, Beucler I, Bruckert E, Chambaz J, Chanu B, Lecerf J, Luc G, Moulin P, Weissenbach J, Prat A, Krempf M, Junien C, Seidah N, Boileau C (2003). Mutations in PCSK9 cause autosomal dominant hypercholesterolemia. Nat. Genet 34, 154-156.
3. Pisciotta L, Priore Oliva C, Cefalu AB, Noto D, Bellocchio A, Fresa R, Cantafora A, Patel D, Averna M, Tarugi P, Calandra S, Bertolini S (2006). Additive effect of mutations in LDLR and PCSK9 genes on the phenotype of familial hypercholesterolemia. Atherosclerosis 186, 433-440.
4. Maxwell K, Breslow J (2004). Adenoviral-mediated expression of PCSK9 in mice results in a low-density lipoprotein receptor knockout phenotype. Proc Natl Acad Sci USA 101, 7100-7105.
5. Benjannet S, Rhainds D, Essalmani R, Mayne J, Wickham L, Jin W, Asselin M, Hamelin J, Varret M, Allard D, Trillard M, Abifadel M, Tebon A, Attie AD, Rader DJ, Boileau C, Brissette L, Chrétien M, Prat A, Seidah NG (2004). NARC-1/PCSK9 and its natural mutants: zymogen cleavage and effects on the low density lipoprotein (LDL) receptor and LDL cholesterol. J Biol Chem 279, 48865-48875.
6. Cohen J, Pertsemlidis A, Kotowski I, Graham R, Garcia C, Hobbs H (2005). Low LDL cholesterol in individuals of African descent resulting from frequent nonsense mutations in PCSK9. Nature Genetics 37, 161-165.
7. Rashid S, Curtis D, Garuti R, Anderson N, Bashmakov Y, Ho Y, Hammer H, Moon Y, Horton J (2005). Decreased plasma cholesterol and hypersensitivity to statins in mice lacking PCSK9. Proc Natl Acad Sci USA 102, 5374-5379.
8. Zhao Z, Tuakli-Wosornu Y, Lagace T, Kinch L, Grishin N, Horton J, Cohen J, Hobbs H (2006). Molecular Characterization of Loss-of-Function Mutations in PCSK9 and Identification of a Compound Heterozygote. Am J Human Genetics 79, 514-523.
9. Benjannet S, Rhainds D, Hamelin J, Nassoury N, Seidah NG (2006). The proprotein convertase PCSK9 is inactivated by furin and/or PC5/6A: functional consequences of natural mutations and post-translational modifications. J Biol Chem 281, 30561-30572.
10. Li J, Tumanut C, Gavigan J-A, Huang W-J, Hampton EN, Tumanut R, Suen KF, Trauger JW, Spraggon G, Lesley SA, Liau G, Yowe D, Harris JL (2007). Secreted PCSK9 promotes LDL receptor degradation independently of proteolytic activity. Biochem J 406, 203-207.
11. McNutt MC, Lagace TA, Horton JD (2007). Catalytic activity is not required for secreted PCSK9 to reduce low density lipoprotein receptors in HepG2 cells. J Biol Chem 282, 20799-20803.
12. Zhang D-W, Lagace TA, Garuti R, Zhao Z, McDonald M, Horton JD, Cohen JC, Hobbs HH (2007). Binding of proprotein convertase subtilisin/kexin type 9 to epidermal growth factor-like repeat A of low density lipoprotein receptor decreases receptor recycling and increases degradation. J Biol Chem 282, 18602-18612.
13. Kwon HJ, Lagace TA, McNutt MC, Jay D. Horton JD, Deisenhofer J (2008). Molecular basis for LDL receptor recognition by PCSK9. Proc Natl Acad Sci USA 105, 1820-5.
14. Bottomley MJ, Cirillo A, Orsatti L, Ruggeri L, Fisher TS, Santoro JC, Cummings RT, Cubbon RM, Lo Surdo P, Calzetta A, Noto A, Baysarowich J, Mattu M, Talamo F, De Francesco R, Sparrow CP, Sitlani A, Carfi A (2009). Structural and biochemical characterization of the wild type PCSK9/EGF(AB) complex and natural familial hypercholesterolemia mutants. J Biol Chem 284, 1313-1323.
15. Seidah NG (2009). PCSK9 as a therapeutic target of dyslipidemia. Expert Opin Ther Targets 13, 19-28.
16. Graham MJ, Lemonidis KM, Whipple CP, Subramaniam A, Monia BP, Crooke ST, Crooke RM (2007). Antisense inhibition of proprotein convertase subtilisin/kexin type 9 reduces serum LDL in hyperlipidemic mice. J Lipid Res 48, 763-767.
17. Frank-Kamenetsky M, Grefhorst A, Anderson NN, Racie TS, Bramlage B, Akinc A, Butler D, Charisse K, Dorkin R, Fan Y, Gamba-Vitalo C, Hadwiger P, Jayaraman M, John M, Jayaprakash KN, Maier M, Nechev L, Rajeev KG, Read T, Röhl I, Soutschek J, Tan P, Wong J, Wang G, Zimmermann T, de Fougerolles A, Vornlocher HP, Langer R, Anderson DG, Manoharan M, Koteliansky V, Horton JD, Fitzgerald K (2008). Therapeutic RNAi targeting PCSK9 acutely lowers plasma cholesterol in rodents and LDL cholesterol in nonhuman primates. Proc Natl Acad Sci USA 105, 11915-11920.
18. Piper D, Jackson S, Liu Q, Romanow W, Shetterly S, Thibault S, Shan B, Walker N (2007). The Crystal Structure of PCSK9: A Regulator of Plasma LDL-Cholesterol. Structure 15, 545-552.
19. Cunningham D, Danley DE, Geoghegan KF, Matthew C Griffor MC, Hawkins JL, Subashi TA, Varghese AH, Ammirati MJ, Culp JS, Hoth LR, Mansour MN, McGrath KM, Seddon AP, Shenolikar S, Stutzman-Engwall KJ, Warren LC, Xia D, Qiu X (2007). Structural and biophysical studies of PCSK9 and its mutants linked to familial hypercholesterolemia. Nature Struc Mol Biol 14, 413-419.
20. Seidah N, Benjannet S, Wickham L, Marcinkiewicz J, Jasmin S, Stifani S, Basak A, Prat A, Chrétien M (2003). The secretory proprotein convertase neural apoptosis-regulated convertase 1 (NARC-1) liver regeneration and neuronal differentiation. Proc Natl Acad Sci USA 100, 928-933.
21. McNutt MC, Kwon HJ, Chen C, Chen JR, Horton JD, Lagace TA (2009). Antagonism of secreted PCSK9 increases low density lipoprotein receptor expression in HepG2 cells. J Biol Chem 284, 10561-10570.
22. Swergold G, Biedermann S, Renard R, Nadler D, Wu R; Mellis S (2010). Safety, Lipid, and Lipoprotein Effects of REGN727/SAR236553, a Fully-Human Proprotein Convertase Subtilisin Kexin 9 (PCSK9) Monoclonal Antibody Administered Intravenously to Healthy Volunteers. Circulation 122, A23251.
23. Dias C, Shaywitz A, Smith B, Emery M, Bing G, Gibbs J, Wishner B, Stolman D, Crispino C, Cook B, Colbert A, Retter M, Xu R (2011). A Phase 1, Randomized, Double-Blind, Placebo-Controlled, Ascending Single Dose Study to Evaluate the Safety, Tolerability and Pharmacodynamics of AMG145. Circulation 124.
24. Amgen (2010) Ascending Multiple Dose Study to Evaluate the Safety, Tolerability, Pharmacokinetics and Pharmacodynamics of AMG 145 in Subjects With Hyperlipidemia on Stable Doses of a Statin. ClinicalTrails.Gov.
25. Crunkhorn S (2012). PCSK9 antibody reduces LDL cholesterol. Nature Rev Drug Disc 11, 11.

## Claims

1. A compound for use in a method for treating or preventing hypercholesterolemia, and/or at least the symptom of hypercholesterolemia in dyslipidemia, atherosclerosis, CVD or coronary heart disease, wherein the compound is represented by the formula: 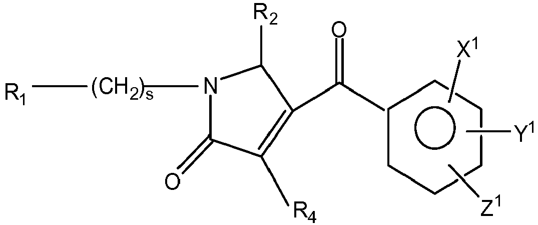
wherein R₁ is selected from the group of optionally substituted cycloalkyl, cycloalkyloxy, aryl, aryloxy, aralkoxy, heterocycle, heterocycloalkoxy, heteroaryl, and heteroaralkoxy; R₂ is selected from the group of H and optionally substituted lower alkyl, cycloalkyl, aryl, heterocycle, and heteroaryl;
R₄ is selected from the group of H, lower alkyl, OH, SH, NH₂, halo, CN, carboxyl, and carboxamido;
wherein X¹, Y¹ and Z¹ are the same or different and each represents hydrogen or a substituent from the group consisting of hydroxyl, halogen, amino, monoalkylamino, dialkylamino, alkoxy, carboxy, amido (including formamido, alkylamido and arylamido), aminocarbonyl, monoalkylaminocarbonyl, dialkylaminocarbonyl, carbamato, carboxamido, monoalkylaminosulfinyl, dialkylaminosulfinyl, monoalkylaminosulfonyl, dialkylaminosulfonyl, alkylsulfonylamino, hydroxysulfonyloxy, alkoxysulfonyloxy, alkylsulfonyloxy, hydroxysulfonyl, alkoxysulfonyl, alkylsulfonylalkyl, monoalkylaminosulfonylalkyl, dialkylaminosulfonylalkyl, monoalkylaminosulfinylalkyl, dialkylaminosulfinylalkyl and, optionally substituted, lower alkyl, alkenyl, alkynyl, cycloalkyl, aryl, heterocycle, and heteroaryl;
and s is an integer from 1 to 4;
or the pharmaceutically acceptable salts and all stereoisomers of said compound.

2. The compound for use in a method of treatment of claim 1, wherein said compound is selected from the group consisting of:
SBC-115,076: 4-[4-(benzyloxy)-3-methylbenzoyl]-3-hydroxy-1-[3-(morpholin-4-yl)propyl]-5-(pyridin-4-yl)-2,5-dihydro-1H-pyrrol-2-one
SBC-115,081: 1-[3-(dimethylamino)propyl]-3-hydroxy-5-(3-hydroxyphenyl)-4-{4-[(3-methylphenyl)methoxy]benzoyl}-2,5-dihydro-1H-pyrrol-2-one
SBC-115,084: 3-hydroxy-5-(3-hydroxyphenyl)-1-[3-(1H-imidazol-1-yl)propyl]-4-{4-[(2-methylphenyl)methoxy]benzoyl}-2,5-dihydro-1H-pyrrol-2-one
SBC-115,086: 5-(3-chlorophenyl)-3-hydroxy-4-(7-methoxy-1-benzofuran-2-carbonyl)-1-[3-(morpholin-4-yl)propyl]-2,5-dihydro-1H-pyrrol-2-one
SBC-115,177: 3-hydroxy-4-{4-[(3-methylphenyl)methoxy]benzoyl}-1-[3-(morpholin-4-yl)propyl]-5-(pyridin-4-yl)-2,5-dihydro-1H-pyrrol-2-one
SBC-115,178: 4-[4-(benzyloxy)-2-methylbenzoyl]-3-hydroxy-1-[3-(morpholin-4-yl)propyl]-5-(pyridin-3-yl)-2,5-dihydro-1H-pyrrol-2-one
SBC-115,179: 4-[4-(benzyloxy)-3-methylbenzoyl]-3-hydroxy-1-[3-(morpholin-4-yl)propyl]-5-(thiophen-2-yl)-2,5-dihydro-1H-pyrrol-2-one
SBC-115,181: 4-[4-(benzyloxy)-3-methylbenzoyl]-3-hydroxy-1-[3-(morpholin-4-yl)propyl]-5-(pyridin-3-yl)-2,5-dihydro-1H-pyrrol-2-one.

3. The compound for use in a method of treatment of claim 1, wherein said compound is 4-[4-(benzyloxy)-3-methylbenzoyl]-3-hydroxy-1-[3-(morpholin-4-yl)propyl]-5-(pyridin-4-yl)-2,5-dihydro-1H-pyrrol-2-one (SBC-115,076):

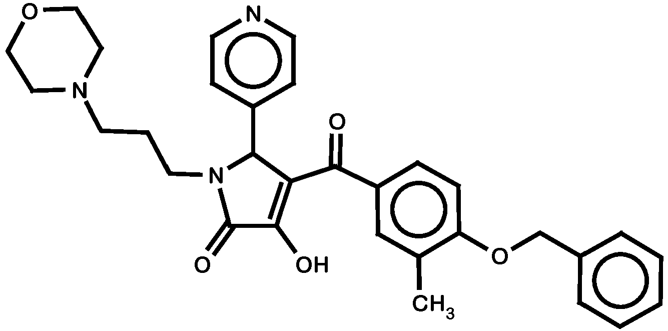

## Patentansprüche

1. Verbindung zur Verwendung in einem Verfahren zur Behandlung oder Vorbeugung von Hypercholesterinämie und/oder zumindest des Symptoms der Hypercholesterinämie bei Dyslipiämie, Atherosklerose, CVD oder koronarer Herzkrankheit, wobei die Verbindung durch die folgende Formel dargestellt ist: 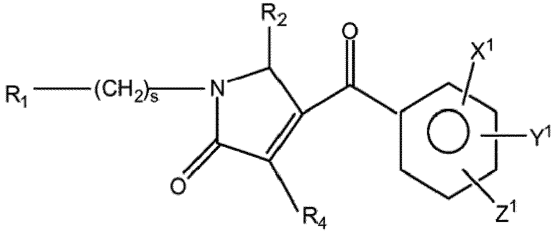
worin R₁ aus der Gruppe aus gegebenenfalls substituiertem Cycloalkyl, Cycloalkyloxy, Aryl, Aryloxy, Aralkoxy, Heterocyclyl, Heterocycloalkoxy, Heteroaryl und Heteroaralkoxy ausgewählt ist;
R₂ aus der Gruppe aus H und gegebenenfalls substituiertem Niederalkyl, Cycloalkyl, Aryl, Heterocyclyl und Heteroaryl ausgewählt ist;
R₄ aus der Gruppe aus H, Niederalkyl, OH, SH, NH₂, Halogen, CN, Carboxyl und Carboxamido ausgewählt ist;
worin X¹, Y¹ und Z¹ gleich oder unterschiedlich sind und jeweils für Wasserstoff oder einen Substituenten aus der aus Hydroxyl, Halogen, Amino, Monoalkylamino, Dialkylamino, Alkoxy, Carboxy, Amido (einschließlich Formamido, Alkylamido und Arylamido), Aminocarbonyl, Monoalkylaminocarbonyl, Dialkylaminocarbonyl, Carbamato, Carboxamido, Monoalkylaminosulfinyl, Dialkylaminosulfinyl, Monoalkylaminosulfonyl, Dialkylaminosulfonyl, Alkylsulfonylamino, Hydroxysulfonyloxy, Alkoxysulfonyloxy, Alkylsulfonyloxy, Hydroxysulfonyl, Alkoxysulfonyl, Alkylsulfonylalkyl, Monoalkylaminosulfonylalkyl, Dialkylaminosulfonylalkyl, Monoalkylaminosulfinylalkyl, Dialkylaminosulfinylalkyl und gegebenenfalls substituiertem Niederalkyl, Alkenyl, Alkinyl, Cycloalkyl, Aryl, Heterocyclyl und Heteroaryl bestehenden Gruppe stehen;
und s eine ganze Zahl von 1 bis 4 ist;
oder die pharmazeutisch annehmbaren Salze und alle Stereoisomere der Verbindung.

2. Verbindung zur Verwendung in einem Behandlungsverfahren nach Anspruch 1, worin die Verbindung ausgewählt ist aus der Gruppe bestehend aus:
SBC-115,076: 4-[4-(Benzyloxy)-3-methylbenzoyl]-3-hydroxy-1-[3-(morpholin-4-yl)-propyl]-5-(pyridin-4-yl)-2,5-dihydro-1H-pyrrol-2-on;
SBC-115,081: 1-[3-(Dimethylamino)propyl]-3-hydroxy-5-(3-hydroxyphenyl)-4-{4-[(3-methylphenyl)methoxy]benzoyl}-2,5-dihydro-1H-pyrrol-2-on;
SBC-115,084: 3-Hydroxy-5-(3-hydroxyphenyl)-1-[3-(1H-imidazol-1-yl)propyl]-4-{4-[(2-methylphenyl)methoxy]benzoyl}-2,5-dihydro-1H-pyrrol-2-on;
SBC-115,086: 5-(3-Chlorphenyl)-3-hydroxy-4-(7-methoxy-1-benzofuran-2-carbonyl)-1-[3-(morpholin-4-yl)propyl]-2,5-dihydro-1H-pyrrol-2-on;
SBC-115,177: 3-Hydroxy-4-{4-[(3-methylphenyl)methoxy]benzoyl}-1-[3-(morpholin-4-yl)propyl]-5-(pyridin-4-yl)-2,5-dihydro-1H-pyrrol-2-on;
SBC-115,178: 4-[4-(Benzyloxy)-2-methylbenzoyl]-3-hydroxy-1-[3-(morpholin-4-yl)-propyl]-5-(pyridin-3-yl)-2,5-dihydro-1H-pyrrol-2-on;
SBC-115,179: 4-[4-(Benzyloxy)-3-methylbenzoyl]-3-hydroxy-1-[3-(morpholin-4-yl)-propyl]-5-(thiophen-2-yl)-2,5-dihydro-1H-pyrrol-2-on;
SBC-115,181 : 4-[4-(Benzyloxy)-3-methylbenzoyl]-3-hydroxy-1-[3-(morpholin-4-yl)-propyl]-5-(pyridin-3-yl)-2,5-dihydro-1H-pyrrol-2-on.

3. Verbindung zur Verwendung in einem Behandlungsverfahren nach Anspruch 1, wobei die Verbindung 4-[4-(Benzyloxy)-3-methylbenzoyl]-3-hydroxy-1-[3-(morpholin-4-yl)propyl]-5-(pyridin-4-yl)-2,5-dihydro-1H-pyrrol-2-on (SBC-115,076) ist:

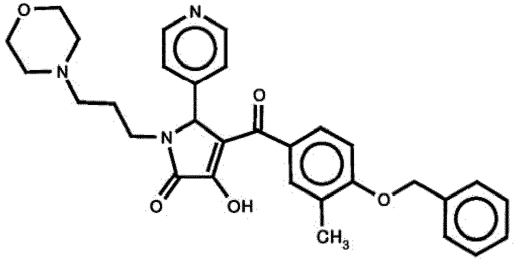

## Revendications

1. Composé pour une utilisation dans une méthode de traitement ou de prévention de l'hypercholestérolémie, et/ou au moins du symptôme d'hypercholestérolémie dans la dyslipidémie, l'athérosclérose, les MCV ou les maladies coronariennes, lequel composé est le composé représenté par la formule : 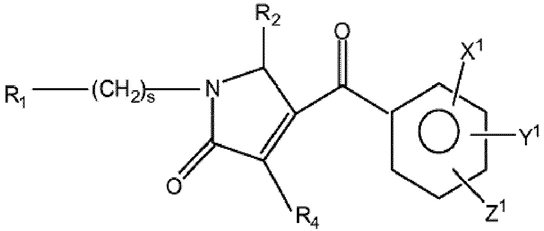
dans laquelle R₁ est choisi dans l'ensemble constitué par les radicaux éventuellement substitués cycloalkyle, cycloalkyloxy, aryle, aryloxy, aralcoxy, hétérocycliques, hétérocycloalcoxy, hétéroaryle, et hétéroaralcoxy ;
R₂ est choisi dans l'ensemble constitué par H et les radicaux éventuellement substitués alkyle inférieur, cycloalkyle, aryle, hétérocycliques, et hétéroaryle ;
R₄ est choisi dans l'ensemble constitué par H et les radicaux alkyle inférieurs, OH, SH, NH₂, halogéno, CN, carboxyle, et carboxamido ;
dans laquelle chacun de X¹, Y¹ et Z¹, qui sont identiques ou différents, représente l'hydrogène ou un substituant de l'ensemble constitué par les halogènes, les radicaux hydroxyle, amino, monoalkylamino, dialkylamino, alcoxy, carboxy, amido (y compris formamido, alkylamido et arylamido), aminocarbonyle, monoalkylaminocarbonyle, dialkylaminocarbonyle, carbamato, carboxamido, monoalkylaminosulfinyle, dialkylaminosulfinyle, monoalkylaminosulfonyle, dialkylaminosulfonyle, alkylsulfonylamino, hydroxysulfonyloxy, alcoxysulfonyloxy, alkylsulfonyloxy, hydroxysulfonyle, alcoxysulfonyle, alkylsulfonylalkyle, monoalkylaminosulfonylalkyle, dialkylaminosulfonylalkyle, monoalkylaminosulfinylalkyle, dialkylaminosulfinylalkyle, et les radicaux éventuellement substitués alkyle inférieur, alcényle, alcynyle, cycloalkyle, aryle, hétérocycliques, et hétéroaryle ;
et s est un entier de 1 à 4 ;
ou les sels pharmaceutiquement acceptables et tous les stéréoisomères dudit composé.

2. Composé pour une utilisation dans une méthode de traitement selon la revendication 1, lequel composé est choisi dans l'ensemble constitué par les suivants :
SBC-115,076 : 4-[4-(benzyloxy)-3-méthylbenzoyl]-3-hydroxy-1-[3-(morpholin-4-yl)propyl]-5-(pyridin-4-yl)-2,5-dihydro-1H-pyrrol-2-one
SBC-115,081 : 1-[3-(diméthylamino)propyl]-3-hydroxy-5-(3-hydroxyphényl)-4-{4-[(3-méthylphényl)méthoxy]benzoyl}-2,5-dihydro-1H-pyrrol-2-one
SBC-115,084 : 3-hydroxy-5-(3-hydroxyphényl)-1-[3-(1H-imidazol-1-yl)propyl]-4-{4-[(2-méthylphényl)méthoxy]benzoyl}-2,5-dihydro-1H-pyrrol-2-one
SBC-115,086 : 5-(3-chlorophényl)-3-hydroxy-4-(7-méthoxy-l-benzofurane-2-carbonyl)-1-[3-(morpholin-4-yl)propyl]-2,5-dihydro-1H-pyrrol-2-one
SBC-115,177 : 3-hydroxy-4-{4-[(3-méthylphényl)méthoxy]benzoyl}-1-[3-(morpholin-4-yl)propyl]-5-(pyridin-4-yl)-2,5-dihydro-1H-pyrrol-2-one
SBC-115,178 : 4-[4-(benzyloxy)-2-méthylbenzoyl]-3-hydroxy-1-[3-(morpholin-4-yl)propyl]-5-(pyridin-3-yl)-2,5-dihydro-1H-pyrrol-2-one
SBC-115,179 : 4-[4-(benzyloxy)-3-méthylbenzoyl]-3-hydroxy-1-[3-(morpholin-4-yl)propyl]-5-(thiophén-2-yl)-2,5-dihydro-1H-pyrrol-2-one.
SBC-115,181 : 4-[4-(benzyloxy)-3-méthylbenzoyl]-3-hydroxy-1-[3-(morpholin-4-yl)propyl]-5-(pyridin-3-yl)-2,5-dihydro-1H-pyrrol-2-one.

3. Composé pour une utilisation dans une méthode de traitement selon la revendication 1, lequel composé est la 4-[4-(benzyloxy)-3-méthylbenzoyl]-3-hydroxy-1-[3-(morpholin-4-yl)propyl]-5-(pyridin-4-yl)-2,5-dihydro-1H-pyrrol-2-one (SBC-115,076) :
